# EUROPEAN PATENT SPECIFICATION

(11) **EP 1 978 903 B1**
(45) Date of publication and mention of the grant of the patent: **25.07.2012**
(21) Application number: 06809986.0
(22) Date of filing: 20.09.2006
(51) Int. Cl.: A61F 5/01

(54) **CONTROL DEVICE FOR ADJUSTABLE -ANGLE ORTHOPEDIC BRACES**
KONTROLLVORRICHTUNG FÜR KIEFERORTHOPÄDISCHE SPANGEN MIT VERSTELLBAREM WINKEL
DISPOSITIF DE REGLAGE POUR ORTHESE A ANGLE AJUSTABLE

(30) Priority: 26.10.2005 IT VR20050135
(43) Date of publication of application: 15.10.2008
(73) Proprietor: F.G.P. Srl, 37062 Dossobuono (VR) (IT)
(72) Inventor: TURRINI, Alberto, I-37062 CASTEL D1AZZANO (VR) (IT)
(74) Representative: Sandri, Sandro
(86) International application number: PCT/IT2006/000671
(87) International publication number: WO 2007/049318

(56) References cited:
- EP-A2- 0 144 479
- WO-A-91/12786
- WO-A-97/00661
- US-A- 4 494 534
- US-A- 4 624 247
- US-A- 5 460 599

## Description

### TECHNICAL FIELD

This invention concerns a control device for recording and displaying the angular excursions of adjustable-angle orthopedic braces.

More specifically, the invention refers to a control device designed to be fitted to articulated joints for the knee or for other joints, such as the elbow or the ankle, with adjustable angular excursion.

The control device according to the invention acts as an interface between the articulated joint of a brace, that can be fitted during sports activities and post-operative rehabilitation, and the subject who must wear it for certain periods.

The articulated joint can also be fitted in order to control tibial pathological rotation, translation and hypertension.

This invention can be applied in the health-medical industry and specifically in the production sector of orthopedic instruments and accessories in general, as well as of prostheses and braces mainly used in conservative, post-traumatic, rehabilitation and post-operative treatment.

### BACKGROUND ART

It is known that for some orthopedic disorders, particularly those involving the knee, the elbow or the ankle, such as following injury to the ligaments or other parts of the joints or following surgery, it is necessary to use a knee brace or prosthesis for the leg or the arm that is designed to act as a hinged restraint, for example between the femur and the tibia in the leg, supporting most of the stress that would otherwise be harmful for the rehabilitation of the knee.

We will consider the example of the knee joint, and thus of knee braces, although it is understood that the same concept can be applied to other braces, for example for the elbow, the ankle or other parts of the arms or legs.

Knee braces and other braces for the arms and legs usually consist of a rigid frame that encircles the limb at the level of the joint in order to relieve the joint of all the stress that would otherwise be harmful when the injured and/or convalescent subject walks.

The frame of the knee brace or other similar brace generally comprises means of restraint to the femur and the tibia in zones close to the knee and a structure which connects these means consisting of a hinge positioned at the level of the knee.

According to the known technique, an articulated joint for a knee brace usually consists of a pair of reciprocally hinged discs, each attached to a respective upright which is in turn fixed to the limb of the user.

Since it is indispensable in post-traumatic therapy for the angular excursion between the discs to be limited to within a range that gradually increases over time, and a different degree of control must be ensured for the angular excursion during extension with respect to the angular excursion during flexion, the articulated joint must foresee adjustment devices that are easily accessible as well as easily maneuvrable.

According to the known solutions, traditional articulated joints comprise a pair of outer discs connected to a femoral upright and hinged to an intermediate disc connected to the tibial upright, where each outer disc presents at least one toothed circumferential slot designed to accommodate a pair of cursors, in which these cursors can be radially moved inside these outer discs, in opposition to elastic loading means positioned between the said cursor and the central rotation pin of the articulated joint.

It has been found that the adjustment of such an articulated joint for orthopedic braces creates some difficulties for specialized orthopedic staff and, according to precise indications of family doctors, also for the user in cases where it is possible for the user to personally adjust the brace.

All the solutions described which foresee the use of orthopedic braces fitted with an articulated joint, particularly knee braces, are limited in functionality, first of all because they do not foresee sufficiently precise means of adjustment.

In fact, adjustment of the means that control angular excursion is entrusted to very approximate reference elements, being represented by notches or incisions that are often difficult to see and do not allow the necessary precision in adjusting the brace in its various extension and flexion positions.

This is particularly disadvantageous if one considers that a brace should instead be adjusted according to precise angular excursions that should remain limited within a range that gradually increases over time.

This is why the articulated joint for orthopedic braces as they are currently conceived are inadequate for the correct rehabilitation of the joint to be treated.

Document WO 91/12786 A discloses a system for limiting the Range of Motion (ROM) of an articulation including a series of sensors and a control unit aiming to measure the FORCE exerted by a patient while performing an "isometric" 10 exercise. The description of this document highlights how this system allows these exercises to be carried out at different levels of flexion/extension. As a matter of fact, this opportunity is allowed by the presence of an electromechanical joint, that can be oriented in an incremental way, controlled by the control unit that governs the relative position of the two uprights.

### DESCRIPTION OF THE INVENTION

This invention proposes to provide an adjustable-angle control device designed for recording and displaying the angular excursions of orthopedic braces, in particular braces for the knee joint or any other joint, that can eliminate or at least reduce the problems described above.

In particular, the control device designed for recording and displaying the angular excursions of orthopedic braces according to the invention proposes to overcome the drawbacks caused by the traditional impossibility of precise adjustment of the joint's angular excursions other than by excessively empirical and thus imprecise intervention.

The invention also proposes to provide a control device designed for recording and displaying the angular excursions of orthopedic braces which is easy to construct so as to be economically advantageous as well as extremely efficient from the point of view of versatility.

Another advantage proposed by the invention is to provide a control device designed for recording and displaying the angular excursions of orthopedic braces that is equipped with means that are suitable for the visual control and, if necessary, the recording of information relative to the amplitude and the progress of the movements made during the angular excursion of the knee during rehabilitation.

This is achieved by means of a control device designed for the management and setting of the angular excursions of orthopedic braces with the features described in the main claim.

The dependent claims describe advantageous embodiments of the invention.

The proposed aims are achieved, according to the invention, by a control device designed for recording and displaying the angular excursions of orthopedic braces, in particular adjustable-angle knee braces, having the features disclosed in claim 1. The dependent claims describe advantageous forms of embodiment of the invention. The control device according to the present invention substantially consists of a recorder, fitted at the level of the joint providing the information and equipped with an LCD type display which on one hand displays the numerical references relative to the position of the adjustment cursors or pins and on the other makes it possible to display various types of information such as the time taken to perform an exercise, the maximum extensions and flexions achieved during the exercise, as well as the dynamic indication of the position of the articulated joint.

The control device according to the invention can be fitted to the articulated joint of the orthopedic brace, that is to say close to the pair of outer discs connected to the femoral upright and hinged to the intermediate disc connected to the tibial upright.

In such an articulated joint on which the control device according to the invention is fitted, each outer disc presents at least one toothed circumferential slot designed to accommodate cursors, generally two, connected to a strip that can be radically moved in opposition to a spring and free to move inside the outer discs in order to adjust the extension and flexion angles of the knee brace.

### DESCRIPTION OF THE DRAWINGS

Other features and advantages of the invention will become evident on reading the following description of one embodiment of the invention, provided as a non-blinding example, with the help of the accompanying drawings in which:

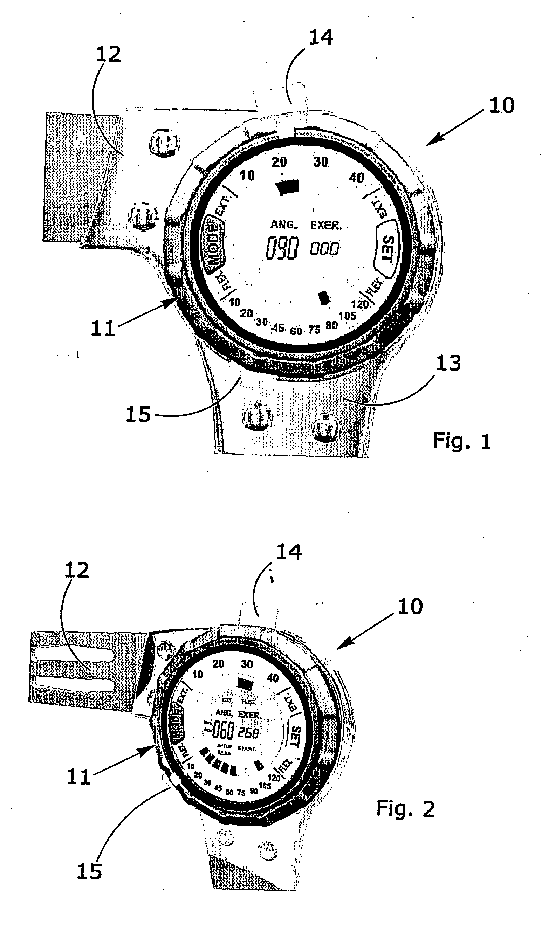

- figure 1 represents the schematic front view of the control device according to the invention, fitted on the articulated joint of a knee brace;
- figure 2 shows the schematic and prospective view of the control device according to the invention, fitted on the articulated joint of a knee brace;
- figure 3 is the schematic front view of a dial with the various types of information displayed;
- figure 4 shows the schematic and prospective view of a possible variation of the control device.

### DESCRIPTION OF ONE EMBODIMENT OF THE INVENTION

The control device designed for recording and displaying the angular excursions of an adjustable-angle orthopedic brace is indicated overall with the number 10, and substantially consists of a dial encircled by a crown 11 and placed over the articulated joint of an orthopedic brace between the femoral upright 12 and the tibial upright 13.

The control device 10 according to the invention can be fitted to the articulated joint of the orthopedic brace, that is to say close to the pair of outer discs connected to the femoral upright 12 and hinged to the intermediate disc connected to the tibial upright.

In such an articulated joint on which the control device according to the invention is fitted, each outer disc presents at least one toothed circumferential slot designed to accommodate two pins 14 and 15, connected to a strip that can be radially moved in opposition to a spring and free to move inside the outer discs in order to adjust the extension and flexion angles of the knee brace.

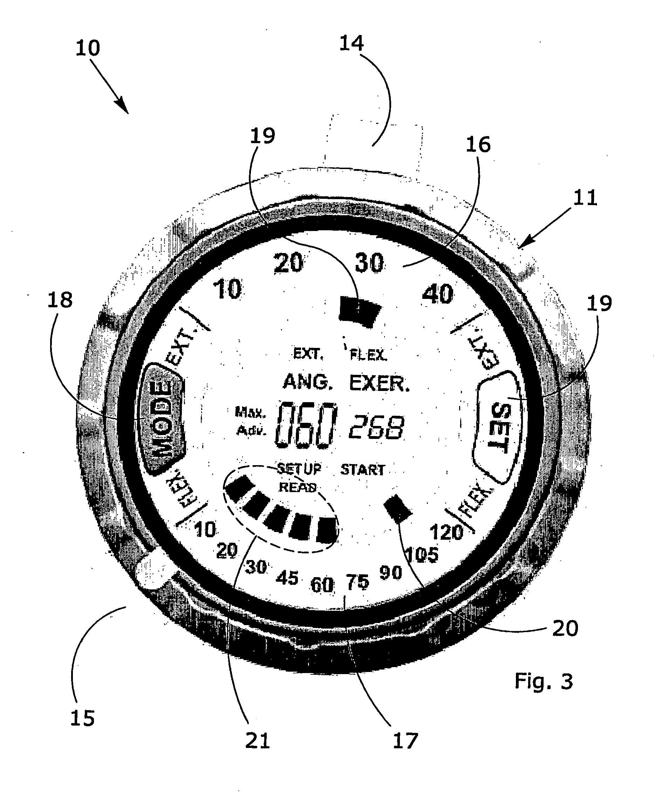

By observing the dial in figure 3, it can be seen that it presents an outer semicircular sector 16 showing the angles in which the extension cursor 14 functions, and an adjacent semicircular sector 17 showing the angles in which the flexion cursor 15 functions.

The outer crown consisting of the semicircular sectors 16 and 17 is made from plastic material or aluminium with printed indications.

The outer circle also includes the control buttons mode 18 and set 19.

Inside the outer circle and occupying the entire inner central zone of the dial is an LCD display. The outer circle of this area includes an indicator 19 showing the pre-set extension limit, adjacent to the indicator 20 showing the pre-set flexion limit.

The sector 21 is a dynamic indicator of the current angular position of the brace.

The centre of the dial includes various types of numerical references relative to the number of extensions and flexions performed, that is to say the instantaneous measurements of the extension and flexion positions and, if required, the maximum numbers reached or the duration of the exercise.

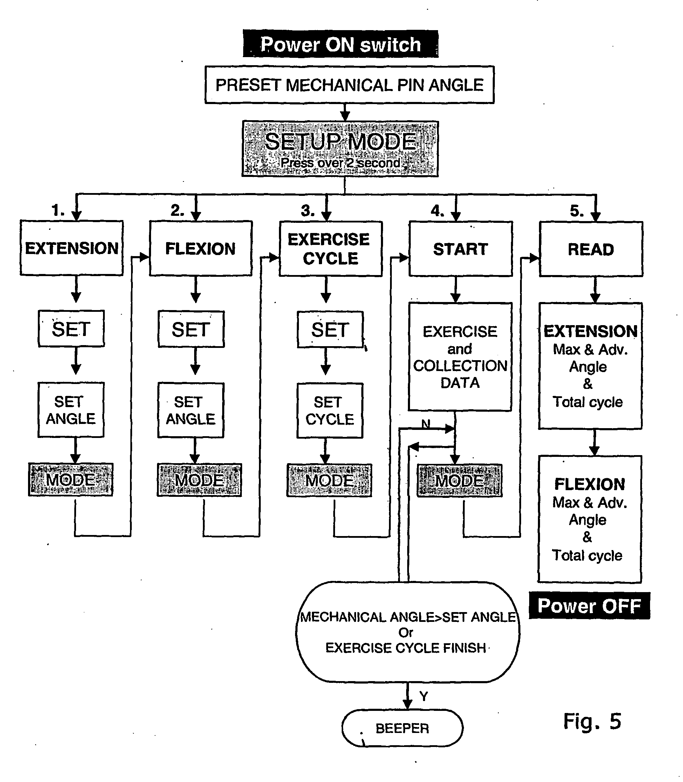

Figure 5 shows a block diagram relative to the functioning and control procedures of the device according to the invention.

In this block diagram it can be seen that the control system manages its data according to the mechanical presetting of the position of each of the two cursors 14 and 15, that is on the basis of their angular position which determines the maximum extension and flexion excursions.

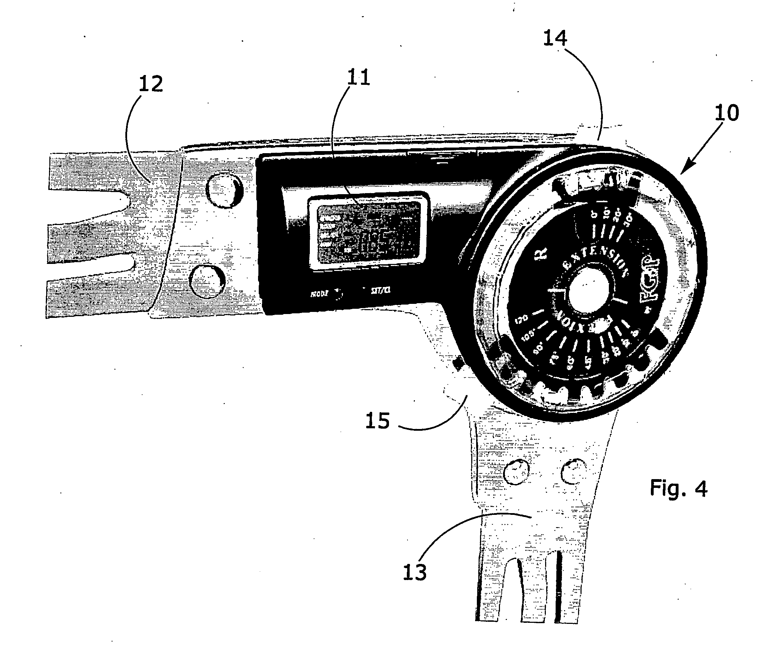

Figure 4 shows another embodiment of the control device according to the invention, considering the possibility that further construction variations can be envisaged.

In this case the numerical display is positioned on one of the two uprights, while the dial with the angular references of the extension and flexion positions is close to the articulated joint.

In any case, the display and its control elements are physically connected to a sensor positioned at the level of the pin of the articulated joint, this sensor being sensitive to the angular movements of the articulated joint.

The display is powered by a source of energy generally consisting of one or more batteries.

Any other construction variation that envisages the use of control devices that can be fitted close to or on the articulated joints of orthopedic braces with adjustable angular excursion may be considered as being within the scope of the invention.

As can be noted, this solution offers the possibility of using a control device designed to record and display the angular excursions of orthopedic braces that is equipped with means for the visual control and for recording the information relative to the amplitude and the progress of the movements made during the angular excursion of the knee, or other joints, during rehabilitation.

## Claims

1. A control device (10) designed to record and display the angular excursions of an adjustable-angle orthopedic brace, this device intended to be fitted at the level of the articulated joint of the orthopedic brace, whereby said articulated joint comprises a pair of outer discs connected to a first upright (12) and hinged to a intermediate disc connected to a second upright (13), each outer disc including a toothed circumferential slot designed to accommodate two adjustment cursors or pins (14, 15) connected to a strip that can be radially moved in opposition to a spring and free to move inside the outer discs in order to adjust the extension and flexion angles of the knee brace, the control device comprises a respective sensor to be positioned in correspondence of a respective pin (14, 15) of the articulated joint, said respective sensor being sensitive to the angular movements of the articulated joint, as well as a recorder, to be fitted at the level of the joint providing the information , **characterized in that** it is further equipped with a display which on one hand displays the numerical data relative to the position of said adjustment cursors or pins (14, 15) and on the other hand makes it possible to display various types of information such as the time taken to perform an exercise, the maximum extensions and flexions achieved during the exercise, as well as the dynamic indication of the position of the articulated joint.

2. A control device (10) according to claim 1, **characterized in that** the display is powered by a source of energy generally consisting of one or more batteries.

3. A control device (10) any one of the preceding claims, **characterized in that** it presents an outer semicircular sector (16) showing the angles in which the extension cursor (14) functions, and an adjacent semicircular sector (17) showing the angles in which the flexion cursor (15) functions.

4. A control device (10) according to any one of the foregoing claims, **characterized in that** the outer circular sector includes mode (18) and set (19) control buttons.

5. A control device (10) according to any one of the foregoing claims, **characterized in that** the central inner zone inside the outer circular sector consists of an LCD display.

6. A control device (10) according to any one of the foregoing claims, **characterized in that** the outer circle of this inner area includes an indicator (19) showing a pre-set extension limit, adjacent to another indicator (20) showing a pre-set flexion limit.

7. A control device (10) according to any one of the foregoing claims, **characterized in that** it comprises a dynamic indicator of the current angular position of the brace.

8. A control device (10) according to any one of the foregoing claims, **characterized in that** close to the centre of the dial are various types of numerical references relative to the number of extensions and flexions performed, that is to say the instantaneous measurements of the extension and flexion positions and, if required, the maximum numbers reached or the duration of the exercise or other information.

## Patentansprüche

1. Steuervorrichtung (10) zur Aufzeichnung und Anzeige der Winkelposition Ausschläge einer Winkel einstellbaren, orthopädischen Orthese, dieses Gerät ist vorgesehen zur Anbringung auf der Ebene des Gelenks der Orthese, wobei die Gelenkverbindung ein Paar von äußeren Lamellen mit einer ersten Stütze (12) und gelenkig mit einer Zwischenscheibe mit einem zweiten Ständer (13) aufweist, wobei jede äußere Scheibe mit einem gezahnten, umfänglichen Schlitz ausgebildet ist, um zwei Einstellungs- Cursor oder Stifte (14, 15) in Eingriff bringbar auszugestalten, verbunden mit einem radial gegen eine Feder bewegbaren Streifen und frei in den äußeren Scheiben zu bewegen, um die Extension und Flexion der Knieorthese einzustellen, das Steuergerät umfasst einen entsprechenden Sensor, positionierbar entsprechend eines jeweiligen Stifts (14, 15) der Gelenkverbindung, wobei der jeweilige Sensor die Winkelbewegungen des Gelenks erfasst, sowie einen Recorder, angebracht auf der Ebene der gemeinsamen Bereitstellung der Informationen, **dadurch gekennzeichnet, dass** sie ferner mit einer Anzeige ausgestattet ist, die einerseits die numerischen Daten bezüglich der Stellung der Einstellelemente Cursor oder Stifte (14, 15) anzeigt und auf der anderen Seite ermöglicht, verschiedene Arten von Informationen anzuzeigen, wie die Zeit für eine Übung, die maximale Streckung und Beugung welche während der Übung erreicht wurde, sowie die dynamische Anzeige der Position des Gelenks.

2. Steuervorrichtung (10) nach Anspruch 1, **dadurch gekennzeichnet, dass** die Anzeige durch eine Energiequelle in der Regel aus einer oder mehreren Batterien betrieben wird.

3. Steuervorrichtung (10) nach einem der vorangehenden Ansprüche, **dadurch gekennzeichnet, dass** sie über einen äußeren halbkreisförmigen Sektor (16), die Winkel der Streckung anzeigend, und einen benachbarten halbkreisförmigen Sektor (17) die Winkel der Beugung anzeigend verfügt.

4. Steuervorrichtung (10) nach einem der vorhergehenden Ansprüche, **dadurch gekennzeichnet, dass** der äußere kreisförmige Sektor Modus- (18) und Einstellungs- (19) Bedientasten umfasst.

5. Steuervorrichtung (10) nach einem der vorhergehenden Ansprüche, **dadurch gekennzeichnet, dass** die zentrale innere Zone innerhalb des äußeren Kreissektors aus einer LCD-Anzeige besteht.

6. Steuervorrichtung (10) nach einem der vorhergehenden Ansprüche, **dadurch gekennzeichnet, dass** der äußere Kreis der inneren Fläche eine Anzeige (19) aufweist, die eine vorgegebene Streckungs- Begrenzung anzeigt, benachbart zu einem anderen Indikator (20), der eine voreingestellte Beugungs-Begrenzung anzeigt.

7. Steuervorrichtung (10) nach einem der vorhergehenden Ansprüche, **dadurch gekennzeichnet, dass** sie einen dynamischen Indikator für die aktuelle Winkelposition der Orthese aufweist.

8. Steuervorrichtung (10) nach einem der vorhergehenden Ansprüche, **dadurch gekennzeichnet, dass** nahe der Mitte des Zifferblattes verschiedene Arten von nummerischen Angaben in Bezug auf die Anzahl von Streckungen und Beugungen angegeben sind, das heißt die augenblickliche Messung der Streckung und Beugung Positionen und, falls gefordert, die maximale Anzahl oder die Dauer der Übung oder andere Informationen.

## Revendications

1. Dispositif de contrôle (10) conçu pour enregistrer et visualiser les excursions angulaires d'un appareil orthopédique à angle réglable, ce dispositif étant destiné à être adapté au niveau de l'articulation de l'appareil orthopédique, ladite articulation comprenant une paire de disques extérieurs connectés à un premier montant (12) et articulés avec un disque intermédiaire connecté à un second montant (13), chaque disque extérieur comportant une fente circonférentielle dentée conçue pour recevoir deux curseurs ou broches de réglage (14, 15) connectés à une bande pouvant être déplacée radialement avec la sollicitation opposée d'un ressort et libres de s'engager dans les disques extérieurs afin de régler les angles d'extension et de flexion de l'appareil de genou, le dispositif de contrôle comprenant un capteur respectif destiné à être placé en correspondance avec une broche respective (14, 15) de l'articulation, ledit capteur respectif étant sensible aux mouvements angulaires de l'articulation, de même qu'un enregistreur, destiné à être adapté au niveau de l'articulation, fournissant les informations, **caractérisé en ce qu'**il est en outre équipé d'un dispositif de visualisation qui, d'une part, permet de visualiser les données numériques relatives à la position desdits curseurs ou broches de réglage (14, 15) et, d'autre part, rend possible de visualiser divers types d'informations, comme le temps mis pour accomplir un exercice, les extensions et flexions maximales réalisées durant l'exercice, de même que l'indication dynamique de la position de l'articulation.

2. Dispositif de contrôle (10) selon la revendication 1, **caractérisé en ce que** le dispositif de visualisation est alimenté par une source d'énergie consistant généralement en une ou plusieurs batteries.

3. Dispositif de contrôle (10) selon l'une quelconque des revendications précédentes, **caractérisé en ce qu'**il présente un secteur semi-circulaire extérieur (16) montrant les angles avec lesquels fonctionne le curseur d'extension (14) et un secteur semi-circulaire adjacent (17) montrant les angles avec lesquels fonctionne le curseur de flexion (15).

4. Dispositif de contrôle (10) selon l'une quelconque des revendications précédentes, **caractérisé en ce que** le secteur circulaire extérieur comporte des boutons de commande de mode (18) et de réglage (19).

5. Dispositif de contrôle (10) selon l'une quelconque des revendications précédentes, **caractérisé en ce que** la zone intérieure centrale à l'intérieur du secteur circulaire extérieur consiste en un dispositif de visualisation à cristaux liquides (LCD).

6. Dispositif de contrôle (10) selon l'une quelconque des revendications précédentes, **caractérisé en ce que** le cercle extérieur de cette zone intérieure comporte un indicateur (19) montrant une limite d'extension préréglée, adjacent à un autre indicateur (20) montrant une limite de flexion préréglée.

7. Dispositif de contrôle (10) selon l'une quelconque des revendications précédentes, **caractérisé en ce qu'**il comprend un indicateur dynamique de la position angulaire actuelle de l'appareil.

8. Dispositif de contrôle (10) selon l'une quelconque des revendications précédentes, **caractérisé en ce que**, près du centre du cadran, sont disposés divers types de références numériques relatives au nombre d'extensions et de flexions accomplies, c'est-à-dire les mesures instantanées des positions d'extension et de flexion et, le cas échéant, les nombres maximum atteints, la durée de l'exercice ou d'autres informations.
